(19) Europäisches Patentamt
European Patent Office
Office européen des brevets

(11) **EP 4 389 915 A1**

(12) **EUROPEAN PATENT APPLICATION**

(43) Date of publication:
**26.06.2024  Bulletin 2024/26**

(21) Application number: **22215708.3**

(22) Date of filing: **21.12.2022**

(51) International Patent Classification (IPC):
***C12Q 1/6883*** (2018.01)

(52) Cooperative Patent Classification (CPC):
**C12Q 1/6883;** C12Q 2600/156; C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ALCEDIAG**
**13790 Peynier (FR)**

(72) Inventors:
• **WEISSMANN, Dinah**
  **34270 SAINT MATHIEU DE TREVIERS (FR)**

• **SALVETAT, Nicolas**
  **34070 MONTPELLIER (FR)**

(74) Representative: **Yes My Patent**
  **32 Boulevard Richard Lenoir**
  **75011 Paris (FR)**

Remarks:
•The complete document including Reference Table(s) and the Sequence Listing(s) can be downloaded from the EPO website
•Claim 16 is deemed to be abandoned due to non-payment of the claims fee (Rule 45(3) EPC).

(54) **DIFFERENTIAL DIAGNOSING OF BIPOLAR VERSUS UNIPOLAR DISORDER IN PATIENT DURING DEPRESSION PHASES USING A TO I RNA EDITING ZNF267 GENE**

(57)    The present invention is drawn to a method for differential diagnosing of bipolar versus unipolar disorder in a human patient during depression phases from a biological sample of said patient, comprising at least the determination of the relative proportion of RNA editing variant (s) of the A to I editing RNA ZNF267 gene. The present invention is also directed to a method for monitoring treatment for depressed patient presenting bipolar or unipolar disorder. Finally, the present invention provides kit for differential diagnosing of bipolar versus unipolar disorder in a patient during depression phases

EP 4 389 915 A1

**Description**

The present invention is drawn to a method for *in* differential diagnosing of bipolar versus unipolar disorder in a human patient during depression phases from a biological sample of said patient, comprising at least the determination of the relative proportion of RNA editing variant (s) of the A to I editing RNA ZNF267 gene. The present invention is also directed to a method for monitoring treatment for depressed patient presenting bipolar or unipolar disorder. Finally, the present invention provides kit for differential diagnosing of bipolar versus unipolar disorder in a patient during depression phases

Depression is one of the most common mental health disorder affecting near 10% of men and 20% of women worldwide, and is associated with a significant increased mortality, mostly due to suicidal behavior. The Diagnostic and Statistics Manual for Mental Disorders characterizes major depressive episode using a combination of five or more different symptoms, e.g. depressed mood, anhedonia, sleep dysregulation, fatigue or indecisiveness. Within mood disorders, bipolar disorder (BD) is one of the most frequent and disabling ones, affecting 1% of the world's population, characterized by episodes of mania, hypomania, and alternating or intertwining episodes of depression. In a primary care clinic, 21% of patients being treated for depression screened positive for BD and 2/3 of them reported that they have never been diagnosed bipolar. Indeed, Judd and colleagues have shown that patients were manic or hypomanic less than 10% of the time and without symptoms about half of the time, meaning that they were depressed during 40% of the time [1]. As a consequence, the average interval between onset of BD symptoms and proper diagnosis is estimated to be around 7 years, delaying suitable treatment and care management, and increasing suicide risk. Various clinical interview-based instruments are available and routinely used in practice by psychiatrists to diagnose BD, including evaluation of manic symptoms by Young Mania Rating Scale (YMRS), the Altman self-rating scale (ASRM)] or the Mood Disorder Questionnaire (MDQ). Biological markers to set the boundaries between the different subtypes of depression are lacking and a major research goal is to identify reliable and clinically useful biomarkers to differentiate BD from unipolar depression.

Recent studies have shown an association between depression and RNA alterations by epitranscriptomic mechanisms [2], including RNA methylation [3], microRNAs [4], and RNA editing [5-7]. One of the most studied processes occurring at the RNA level is the Adenosine (A)-to-inosine (I) conversion, mediated by ADARs (Adenosine deaminase acting on RNA) which bind to double stranded RNA (dsRNA) stem loop and modify A to I by deamination. Inosine is interpreted as guanosine by the cellular machinery due to their similar chemical characteristics. RNA editing can thus induce single amino acid substitutions in coding regions, leading to new start or stop codons or modifying splicing sites. Furthermore, it can also affect RNA stability by modifying the Untranslated Regions UTRs as well as the formation of different microRNAs isoforms. Significant difference in RNA editing has already been reported in neurological or immune diseases, among other pathologies. In the Central Nervous System (CNS), permeability of ion channels and responses to excitatory neurotransmitters have been found to be altered by RNA editing. Recently, we have identified in the anterior cingulate cortex (Brodmann Area 24) of depressed suicide decedents modifications in 5-HTR2c (5-hydroxytryptamine receptor 2c) [8] and PDE8A (PhosphoDiEsterase 8A) [9] mRNA editing. Interestingly, phosphodiesterases, a key modulator of signal transduction downstream 5-HTR2c, is involved in inflammatory cell activation, memory and cognition. The diagnostic value of PDE8A mRNA editing in depression has been also demonstrated, in the blood from HCV patients treated with interferon-$\alpha$ [10], and in the blood from depressed patients and suicide attempters compared to age-matched and sex-matched healthy controls [11].

More recently, Salvetat et al.,[12], have disclosed a study focused on transcriptome-wide RNA editing modifications detected by RNA sequencing (RNA-Seq) to identify new genes with differential A-to-I RNA editing in blood samples from depressed patients as compared to healthy controls (n = 57, discovery cohort). The diagnostic potential of this panel of edited RNA was validated on 410 samples (validation cohort) from either healthy controls (n = 143) or depressed patients (n = 267) by ultra-deep Next Generation Sequencing (NGS). The same approach was applied by dichotomizing the depressed patients in unipolar (n = 160) or bipolar (n = 95) patients to identify specific RNA editing sites or RNA editing isoforms (referred as to biomarkers) on specific RNA sequences (gene targets). The diagnostic performances of their combination was evaluated via a machine learning approach to differentiate unipolar versus BD patients (see also the PCT patent applications WO2021/089865 and WO2021/089866 published on 14-05-2021).

The current COVID-19 pandemic has led populations to lockdown whose demographic and social impacts are still under evaluated. Health restrictions are already causing ravages in the field of psychiatry, with an explosion of anxiety and depression disorders. Indeed, recent studies demonstrate a huge increase of depression prevalence since COVID pandemic [13, 14].

Furthermore, the spread of COVID-19 and important death rate may aggravate the risk of mental health issues and intensify current psychiatric symptoms of certain individuals who are on risk of anxiety, depression, stress, and violence.

It is an object of the present invention to overcome at least one drawback of the prior art. It is moreover the object of the present invention to provide an improved method for the analysis of molecular biomarkers as diagnostic, prognostic and/or predictive aid in the management of depressed patients. It is moreover an object to provide a high sensitivity and robustness improved methods that enable earlier differential diagnosis of bipolar versus unipolar disorder in patient during depression and optionally a reliable prediction of therapy resistance or responsiveness to increase the likelihood of successful treatment.

Thus, the need for a reliable and accurate differential diagnosis, allowing an adequate treatment, of these pathologies has become a crucial necessity for the coming years.

Surprisingly, Zinc finger protein 267 (ZNF267) has been identified as an A to I RNA biomarker exhibiting two major variant types (A-to-G and T-to-C variants) and presenting sites whose editing could be specifically different between unipolar and bipolar disorders, control versus depressive and control versus bipolar disorder (see Table 1).

It has been also demonstrated by the present inventors that using these specific ZNF267 RNA editing sites, isoforms and for the first patterns, or combinations thereof, preferably in combination/association with others A to I editing RNA genes of interest such as GAB2, IFNAR1, LYN, MDM2, PTPRC, IL17RA and PRKCB genes, high diagnostic performance (AUC ROC curve, sensitivity and specificity) on the internal validation cohort (test) and for the first time on an independent cohort (external validation cohort / replication study) have been obtained for differential diagnosis of bipolar versus unipolar patients during depression phases.

Zinc finger protein 267 (ZNF267) is known as a member of the Kruppel-like transcription factor family, which regulates various biological processes such as cell proliferation and differentiation. It has been first characterized as a transcriptional repressor of MMP-10 which is up-regulated during the activation process of human hepatic stellate cells [15]. Zinc finger protein 267 is up-regulated in hepatocellular carcinoma, where it is induced by reactive oxygen species (ROS). Loss and gain of function analysis revealed that ZNF267 promotes tumor cell proliferation and migration of hepatocellular carcinoma cells in vitro [16]. Similarly, expression of ZNF267 in tissue specimens of non-alcoholic fatty liver disease (NAFLD) patients has been found significantly up-regulated compared to normal liver tissue. Incubation of primary human hepatocytes with palmitic acid induced a dose-dependent induction of ZNF267 in vitro, which correlated with lipid accumulation and ROS formation [17]. The similar oncogenic role of ZNF267 in acute lymphoblastic lymphoma (ALL) has been suggested and indicated to be regulated by miRNAs- 23a/b [18]. ZNF267 has been discovered to be hypomethylated in osteoporotic patients, though its role in its role in bone metabolism is still not known [19]. ZNF267 has also been reported to be significantly upregulated during amyloid processing and inflammation [20] ZNF267 have been previously associated with dementia. ZNF267 was selected as one of the ten most predictive genes used in a blood-based transcriptomic panel for diagnosis AD [21]. Recently, ZNF267 has shown to be important transcription factor in the pathogenesis of cognitive impairment and neuroinflammation produced by 1,2-diacetylbenzene (DAB) and targeted by curcumin [22].

This is the object of the present invention.

These objects have been solved by the aspects of the present invention as specified hereinafter.

According to a first aspect, the present invention is directed to a method for analyzing the expression of biomarker RNA molecules is provided, comprising the steps of:

A-isolating RNA from a blood sample obtained from a subject and determining the expression of at least one biomarker RNA molecule in the isolated RNA and providing an expression profile based on the results, wherein said biomarker is the relative proportion of RNA editing at at least a given editing site and/or isoform and/or pattern or a combination of given sites and/or isoforms and/or patterns, which can be edited on ZNF267 gene.

B-from the same isolated RNA, determining the expression of at least another biomarker RNA molecule in the isolated RNA and providing an expression profile based on the results, wherein said biomarker is the relative proportion of RNA editing at at least a given editing site and/or isoform and/or pattern or a combination of given sites and/or isoforms and/or patterns, which can be edited on another A to I editing RNA gene;

and

C-using the expression profiles determined in step A and in B for a combined analysis of the results.

In a preferred embodiment of the present method, in step B, said another A to I editing RNA gene is selected from the group of A to I editing RNA genes consisting of GAB2, IFNAR1, LYN, MDM2, PTPRC, IL17RA and PRKCB A to I editing RNA genes.

In an also preferred embodiment of the present method, in step A, said blood sample is obtained from a patient during depression phase, preferably during moderate or severe depression phases.

In an also preferred embodiment of the present invention, the combined analysis of the results. using the expression profiles determined in step A and in B of the method of the present invention provides a valuable information allowing the differential diagnosis of bipolar disorder versus unipolar disorder in patient during depression, preferably during moderate or severe depression phases of the patient.

As is demonstrated by the examples, a combined analysis of said A to I editing RNA genes biomarkers expression profiles enhances the prognostic and predictive value of the obtained results and can provide valuable diagnostic, prognostic and/or predictive information, particularly for the differential diagnosis of dbipolar disorder versus unipolar disorder in patient during depression, preferably during moderate or severe depression phases of the patient.

The present in vitro method can thus be used as improved diagnostic, prognostic and/or predictive aid in the management of depressed patients. It can be used not only to support the diagnosis, prognosis but also to choose the most appropriate treatment for depressed patients.

According to a second aspect, the present invention is directed to a method for *in vitro* differential diagnosis of bipolar disorder versus unipolar disorder in patient during depression phases preferably during moderate or severe depression phases, the method comprising the determination in a sample of the patient of the relative proportion of RNA editing at at least a given editing site and/or isoform and/or pattern or a combination of given sites and/or isoforms and/or patterns, which can be edited on ZNF267 gene.

By definition, the relative proportion of RNA editing at a given editing 'site' represents the sum of editing modifications measured at this unique genomic coordinate. Conversely, an edited mRNA isoform is a unique molecule that may or may not contain multiple editing modifications on the same transcript. For example for a given transcript, the edited mRNA isoform BC contains an A-to-I modification on both site B and site C within the same transcript. An editing pattern is one combination of editing events occurring at sites of interest in a transcript. By definition, given a list of sites of interest, a transcript has as many editing pattern there are possible combinations. For example for a given transcript, with sites of interest ABC, the analyzed patterns are [A,B,C,AB,BC,AC, ABC].

The terms "biomarker" refers to an editing site or isoform or pattern of RNA that contains one or more positions differently edited, particularly in bipolar versus unipolar disorder comparison.

In a preferred embodiment, said method comprises at list the determination in a sample of the patient of at least the relative proportion of RNA editing of a given pattern or a combination of patterns which can be edited on ZNF267 gene.

In a preferred embodiment, said method according to the present invention further comprises the determination of the relative proportion of RNA editing at at least a given editing site and/or isoform and/or pattern or a combination of given sites and/or isoforms and/or patterns, which can be edited on another one A to I editing RNA gene selected from the group comprising the GAB2, IFNAR1, LYN, MDM2, PTPRC, IL17RA and PRKCB A to I editing RNA genes, more preferably on at least 2, 3, 4, 5 or 6 of these genes.

In an also preferred embodiment, said method comprises at least the determination in a sample of the patient of at least the relative proportion of RNA editing of a given pattern or a combination of patterns which can be edited on GAB2, IFNAR1, LYN, MDM2, PTPRC, IL17RA or PRKCB A to I editing RNA gene.

More preferably, in the method according to the present invention, the determination of the relative proportion of RNA editing at a given editing site and/or isoform and/or pattern or a combination of given sites and/or isoforms and/or patterns, on the A to I ZNF267 gene and on A to I editing RNA gene selected from the group comprising the GAB2, IFNAR1, LYN, MDM2, PTPRC, IL 17RA and PRKCB A to I editing RNA genes, are carried out in the same patient sample.

In a preferred embodiment, said method according to the present invention, further comprised the determination of the relative proportion of RNA editing at a given editing site, and/ or isoform, and/or pattern or combination of given sites, and/or isoforms, and/or patterns which can be edited on the GAB2, IFNAR1, LYN, MDM2, PTPRC, IL17RA and PRKCB A to I editing RNA genes.

In a preferred embodiment the PCR products of the gene region of interest of the selected A to I editing RNA biomarker(s) are sequenced by NGS.

In a preferred embodiment the pair of primers used for obtaining the ZNF267 gene PCR products of interest are:

| | | |
|---|---|---|
| ZNF267 MPx_F2 | GGCTGAGGTGGTCTTGGATG | (SEQ ID No.1) |
| ZNF267 MPx_R1 | CCTCGCCTTCCACTGTGATT | (SEQ ID No.2) |

(see table 2).

In a preferred embodiment the pair of primers used for obtaining the GAB2, IFNAR1, LYN, MDM2, PTPRC, IL17RA and PRKCB gene PCR products of interest are listed in Table 7.

In a preferred embodiment, said method can be also used for in vitro diagnosis bipolar patient in moderate or severe depressed patient.

In another preferred embodiment, said method can be used in vitro diagnosis unipolar patient in moderate or severe depressed patient.

In another preferred embodiment, said method is a method for in vitro diagnosis bipolar patient versus unipolar patient.

The criterion symptoms for bipolar disorders, depression disorders, moderate or severe depression, and MDD are well-known from the skilled person and are for example described in the DSM-5™ book from the American Psychiatric Association (2013, pages DSM-V p123-154);

The diagnoses included in this chapter are bipolar I disorder, bipolar II disorder, cyclothymic disorder, substance/medication-induced bipolar and related disorder, bipolar and related disorder due to another medical condition, other specified bipolar and related disorder, and unspecified bipolar and related disorder. However, the vast majority of individuals whose symptoms meet the criteria for a fully syndromal manic episode also experience major depressive episodes during the course of their lives Bipolar II disorder, requiring the lifetime experience of at least one episode of major depression and at least one hypomania episode, is no longer thought to be a "milder" condition than bipolar I disorder, largely because of the amount of time individuals with this condition spend in depression and because the instability of mood experienced by individuals with bipolar II disorder is typically accompanied by serious impairment in work and social functioning. The diagnosis of cyclothymic disorder is given to adults who experience at least 2 years (for children, a full year) of both hypomania and depressive periods without ever fulfilling the criteria for an episode of mania, hypomania, or major depression.

The criterion symptoms for unipolar depression are also well-known from the skilled person and are for example described in the DSM-5™ book from the American Psychiatric Association (2013, pages 155-188). Depressive disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder.

In another aspect of the present invention, it is provided a method for *in vitro* differential diagnosis of bipolar disorder versus unipolar disorder in patient during depression phases according to the present invention, said method comprising the step of:

a) determining in a sample of the patient to be tested the relative proportion of RNA editing at at least a given editing site and/or isoform and/or pattern, or combination of given sites and/or isoforms and/or patterns, which can be edited on the ZNF267 mRNA gene, and optionally which can be edited on at least another one of the GAB2, IFNAR1, LYN, MDM2, PTPRC, IL17RA and PRKCB A to I editing mRNA gene, preferably 2, 3, 4, 5, 6 or 7 of these genes;.
b) determining a value resulting from the proportion(s) obtained in step a);
c) classifying said patient as being bipolar or unipolar after comparing said resulting value obtained in step b) to a control value obtained for bipolar and/or unipolar patients, wherein said control values were determined in a manner comparable to that of the resulting value obtained in step b).

In a preferred embodiment, in step c) of said methods according to the present invention, said resulting value obtained for the patient to be tested is compared to a threshold associated to the disorder which is desired to be identified (for example, but non-limited to a cut-off or a probability), classifying said patient as being bipolar or unipolar patient according to the chosen threshold if said resulting value is equal to, or greater or smaller than said threshold.

According to a preferred embodiment, the sample of the patient is a biological sample, preferred are blood, serum, urine, saliva, tear fluid or plasma, more preferably blood sample, blood serum, even more preferably the sample of the patient is a blood sample, even more preferably a blood serum sample.

According to yet another preferred embodiment of the present invention, the method is carried out *in vitro* or *ex vivo.*

For example, but non-limited to, in the method of differential diagnosing according to the present invention, said threshold, cut-off or probability can be but non-limited to:

- the Z value calculated for unipolar or bipolar patient during depression phases for the learning cohort

In the present description, the term gene or target have the same meaning and can be used interchangeably.

In a preferred embodiment, in the in vitro method for differential diagnosing bipolar versus unipolar patient during depression phases, according to present invention, the combinations of at least two biomarkers are selected whether said combinations are statistically significant between patients having bipolar or unipolar disorders, with for example a p value $<10^{-4}$.

Are also preferred the in vitro method for differential diagnosing of bipolar versus unipolar patient during depression phases, according to present invention, wherein in step b), the resulting value is calculated by an algorithm implementing a multivariate method including:

- mROC program, particularly to identify the linear combination, which maximizes the AUC (Area Under the Curve) ROC and wherein the equation for the respective combination is provided and can be used as a new virtual marker Z, as follows:

5

$$Z = a.(\text{Biomarker 1}) + b.(\text{Biomarker 2}) + \ldots i.(\text{Biomarker } i) + \ldots n.(\text{Biomarker } n)$$

where i are calculated coefficients and (Biomarker i) are the level of the considered biomarker (i.e. level of RNA editing site or of isoforms for a given target/biomarker); and/or

- a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
- a multivariate analysis applied to assess the RNA editing site(s) and/or isoforms combinations for the diagnostic, said multivariate analysis being selecting for example from the group consisting of:
- Logistic regression model applied for univariate and multivariate analysis to estimate the relative risk of patient at different level of RNA editing site or isoforms values;
- CART (Classification And Regression Trees) approach applied to assess RNA editing site(s) and/or isoforms combinations; and/or
- Support Vector Machine (SVM) approach;
- Artificial Neural Network (ANN) approach;
- Bayesian network approach;
- WKNN (weighted k-nearest neighbours) approach;
- Partial Least Square - Discriminant Analysis (PLS-DA);
- Linear and Quadratic Discriminant Analysis (LDA / QDA), and
- Extratree algorithm (8).
- Any other mathematical method that combines biomarkers.

Preferably the resulting value is calculated by Extratree algorithm.

Are preferred selected additional biomarkers (in addition to the ZNF267 biomarker) wherein the resulting value in step b) is statistically/specifically different from the obtained control result value at $p<0.05$.

Are also preferred the methods of the present invention wherein the following criteria has to be satisfied for the biomarker or for the combination of biomarkers selected in step a):

- the coverage >30;
- AUC>0.6, more preferred AUC>0.8, even more preferred AUC>0.890 ;
- 0.95>FoldChange>1.05, and
- $p<0.05$.

Are also preferred the methods of the present invention wherein the algorithm or equation allowing the calculation of the result value or depression score Z are selected from the Z equations listed in the examples, preferably the equation implemented a combination of the following 8 biomarkers: ZNF267, GAB2, IFNAR1, LYN, MDM2, PTPRC, IL17RA and PRKCB A to I editing RNA biomarkers.

Are also preferred the methods of the present invention, wherein in step a) the relative proportion of RNA editing at a given editing and/or isoform are measuring by NGS in said biological sample.

Are also preferred the methods of the present invention, wherein in step a), the amplicon/nucleic sequence used for the detection of the RNA editing sites and/or the isoforms of the RNA transcript of said biomarker is obtained or obtainable with the set of primers listed in Table 2 and Table 7 for each of the selected biomarkers.

According to another aspect, a method for determining the effectiveness of a therapy in a subject or for predicting or monitoring therapy response in a patient is provided, comprising:

I-analyzing the expression of biomarker RNA molecules according to the method of the present invention, before and/or during and/or after treatment of the patient; and

II-Using the expression profiles determined for a combined analysis of the results.

In another aspect, the present invention is directed to a method for monitoring treatment for bipolar disorder or unipolar disorder in a human patient during depression phases, from a biological sample of said patient, said method comprising:

A) differential diagnosing of bipolar versus unipolar disorder in said human by the method of the present invention before the beginning of the treatment which is desired to be monitored.

B) repeating steps (a) to c) of the method after a period of time during which said patient receives the desired treatment for said diagnosed bipolar or unipolar disorder, in order to obtain a post-treatment result value;
C) comparing the post-treatment result value from step (c) to the result value obtained before treatment.

In another aspect, the present invention is directed to a method for determining whether a patient during depression phases will be a responder to a bipolar or a disorder treatment allowing the patient to be in a euthymic state (state of normal mood) from a biological sample, said method comprising the step of:

A) differential diagnosing of bipolar versus unipolar disorder in said human by the method of the present invention before the beginning of the treatment which is desired to be monitored.
B) repeating steps (a) to c) of the method of the present invention, after a period of time during which said patient receives the desired treatment for said diagnosed bipolar or unipolar disorder, to obtain a post-treatment result value;
C) comparing the post-treatment result value from step (c) to:

- the result value obtained before treatment, and
- to the result value known or obtained for control bipolar or unipolar disorder patients being in an euthymic state, and

classifying said patient as being responder to the treatment if the post-treatment result value obtained in step B) is closer of said euthymic state result value that said result value obtained before treatment.

In another aspect, the present invention is directed to a kit for differential diagnosing of bipolar versus unipolar disorder in a human patient during moderate or severe depression phases, said kit comprising:

1) - optionally, instructions to apply the method for differential diagnosing of bipolar versus unipolar disorder in a human patient during depression phases, in order to obtain a resulting value the analysis of which determining whether said depressed patient presents a bipolar or unipolar disorder; and
2)

a) the pair of primers having the sequence SEQ ID Nos.1 and 2, and
b) at least 1 pair, preferably 2, 3, 4, 5, 6 or the 7 pairs of primers selected from the group of pairs of primers having the sequences SEQ ID Nos.3 and 4, 5 and 6, 7 and 8, 9 and 10, 11 and 12, 13 and 14, and, 15 and 16, more preferably the 7 pairs of primers having the sequences SEQ ID No.3 and 4, 5 and 6, 7 and 8, 9 and 10, 11 and 12, 13 and 14, and, 15 and16.

In a preferred embodiment, the present invention is directed to a kit for differential diagnosing of bipolar versus unipolar disorder in a human patient during moderate or severe depression phases, said kit comprising:

1) - optionally, instructions to apply the method for differential diagnosing of bipolar versus unipolar disorder in a human patient during depression phases, in order to obtain a resulting value the analysis of which determining whether said depressed patient presents a bipolar or unipolar disorder; and
2)

a) a pair of primers allowing to obtain an amplicon including or identical to the amplicon obtainable by the pair of primers having the sequences SEQ ID Nos.1 and 2; and
b) at least 1 pair, preferably 2, 3, 4, 5, 6 or 7 pairs of primers allowing to obtain amplicon(s) including or identical to the amplicon(s) obtainable by the pairs of primers selecting from the group of pair of primers having the sequences SEQ ID Nos.3 and 4, 5 and 6, 7 and 8, 9 and 10, 11 and 12, 13 and 14, and, 15 and 1616, preferably 7 pairs of primers allowing to obtain 7 amplicon(s) including or identical to the 7 amplicons obtainable by the 7 pairs of primers having the sequences SEQ ID Nos.3 and 4, 5 and 6, 7 and 8, 9 and 10, 11 and 12, 13 and 14, and, 15 and 16.

In another preferred embodiment, the present invention is directed to a kit for differential diagnosing of bipolar versus unipolar disorder in a human patient during moderate or severe depression phases, said kit comprising:

1) optionally, instructions to apply the method for differential diagnosing of bipolar versus unipolar disorder in a human patient during depression phases, in order to obtain a resulting value the analysis of which determining whether said depressed patient presents a bipolar or unipolar disorder; and
2)

a) a pair of primers allowing to obtain an amplicon including at least all the given editing site and/or isoform which can be edited on the ZNF267 mRNA gene, each primer of the pair having less than 40, preferably 35, 30 or 25 nucleotides length and comprising at least 10, preferably 12 or 15 consecutive nucleotides of the primers having the sequences SEQ ID Nos. 1 and 2; and

b) at least 1 pair, preferably 2, 3, 4, 5, 6 or 7 pairs of primers, allowing to obtain amplicon(s) including at least all the given editing sites and/or isoforms which can be edited on the mRNA gene selected from the group consisting of IFNAR1, LYN, PRKCB, MDM2, GAB2, IL17RA and PTPRC mRNA gene, and wherein for each elected mRNA gene, the corresponding pair primers have less than 40, preferably 35, 30 or 25 nucleotides length and comprise at least 10, preferably 12 or 15 consecutive nucleotides of the pair primers having the sequences SEQ ID Nos.3 and 4, 5 and 6, 7 and 8, 9 and 10, 11 and 12, 13 and 14, and, 15 and 16,

preferably 7 pairs of primers allowing to obtain 7 amplicon(s) including at least for each amplicon all the given editing sites and/or isoforms which can be edited on the corresponding mRNA gene and wherein the primers of the 7 pair primers have less than 40, preferably 35, 30 or 25 nucleotides length and comprise at least 10, preferably 12 or 15 consecutive nucleotides of the primers of the corresponding pair having the sequences SEQ ID Nos.3 and 4, 5 and 6, 7 and 8, 9 and 10, 11 and 12, 13 and 14, and, 15 and 16, .

The following examples and the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples, Figures and Tables, for which the legends are given herein below.

## BRIEF DESCRIPTION OF THE DRAWINGS

**Figure 1:** STARD flow diagram of Montpellier participants (Study 1&2)

**Figure 2:** STARD flow diagram of LesToises participants (Study 3)

**Figures 3A and 3B:** Overview of our A-I RNA editing analysis. A) overview of workflow for RNA-Seq and editome analysis; B) overview of bioinformatics workflow for Targeted Next Generation Sequencing

**Figure 4:** Receiver operating characteristic curve and diagnostic performances for bipolar depression on both study for model #154

**Figure 5:** Receiver operating characteristic curve and diagnostic performances for bipolar depression on both study for model #284.

Plotted are the probabilities for the correct response of the tested sets using case specific trained random forest models (extraTree model #284). The test dataset on study 2 contains 70 patients from the unipolar group and 28 from bipolar patients. Replication dataset on study3 contains 67 patients from the unipolar group and 28 from bipolar patients. Number of Target used: 8 (GAB2, IFNAR1, LYN, MDM2, PTPRC, IL17RA, ZNF267 and PRKCB ) and number of RNA editing variant used: 22 (extraTree model #284).

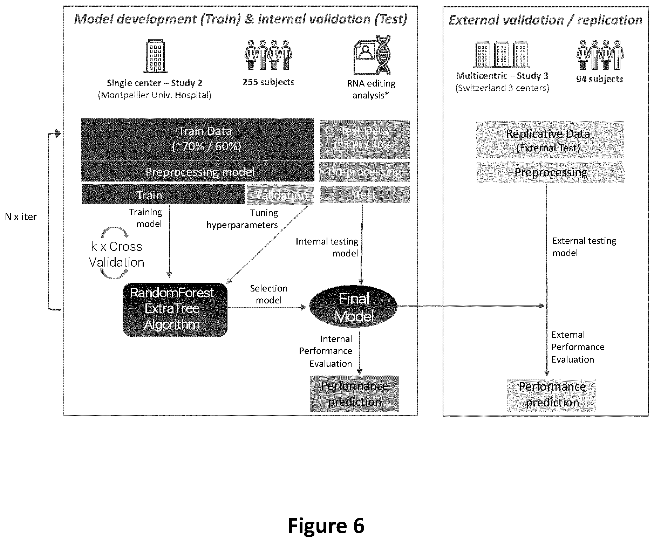

**Figure 6:** Artificial Intelligence procedure

### EXAMPLE 1: Cohort

1.1. Subjects and clinical assessment

### Study 1 (Discovery, CHU Montpellier - France)

Depressed patients (DEP) were recruited from September 2016 to January 2019 among the outpatients of the Department of Emergency Psychiatry and Post-Acute Care (CHRU of Montpellier) according to the principles of the Helsinki Declaration of 1975 and its successive updates. This study was approved by the French local Ethical Committee (CPP Sud-Méditerranée IV in Montpellier, CPP No.A01978-41) and registered under the reference identifer NCT02855918.

A first discovery cohort (n=57) between patients suffering from depression (n=26) and controls (n=31), which was used for RNA-Seq experiments and biomarker discovery. Among the depressed patients, 12 were bipolar.

**Study 2 (Validation, CHU Montpellier** - **France)**

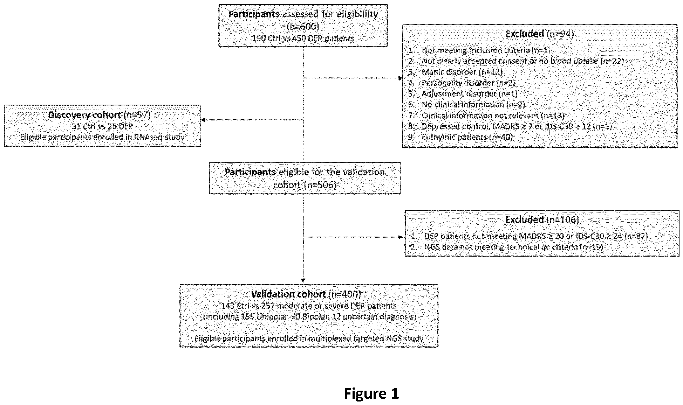

A second validation cohort of 245 caucasian depressed patients (155 unipolar and 90 bipolar) was recruited among the outpatients of the Department of Emergency Psychiatry and Post-Acute Care (CHRU of Montpellier, Clinicaltrials.gov identifier: NCT02855918). This study was approved by the French local Ethical Committee (CPP Sud-Méditerranée IV in Montpellier, CPP No.A01978-41). All participants, aged between 18 and 65 years, understood and signed a written informed consent before entering the study. Diagnosis for unipolar depression and bipolar disorder was made by psychiatrists. During the standardized interview, psychiatrists managed the French version of the Montgomery-Åsberg Depression Rating Scale (MADRS) and the 30-item Inventory of Depressive Symptomatology, Clinician Rated (IDS-C30) to score depression. Depression severity levels, i.e. mild, moderate and severe were defined by $7 \leq MADRS \leq 19$ and/or $12 \leq IDSC-30 \leq 23$, $20 \leq MADRS \leq 34$ and/or $24 \leq IDSC-30 \leq 36$, $MADRS \geq 35$ and/or $IDSC-30 \geq 37$, respectively. Manic symptoms were evaluated by the Young Mania Rating Scale (YMRS). Presence of Bipolar Disorder was assessed by clinician's expertise. All the patients received a treatment classified into these 5 categories: anxiolytics, hypnotics_and_sedatives, antidepressants, antipsychotics, and antiepileptics. The complete flow diagram of the patients through the study is shown in Figure 1(Figure1 : STARD flow diagram of Montpellier participants (Study 1&2))

**Study 3 (Replication, Les Toises center** - **Switzerland)**

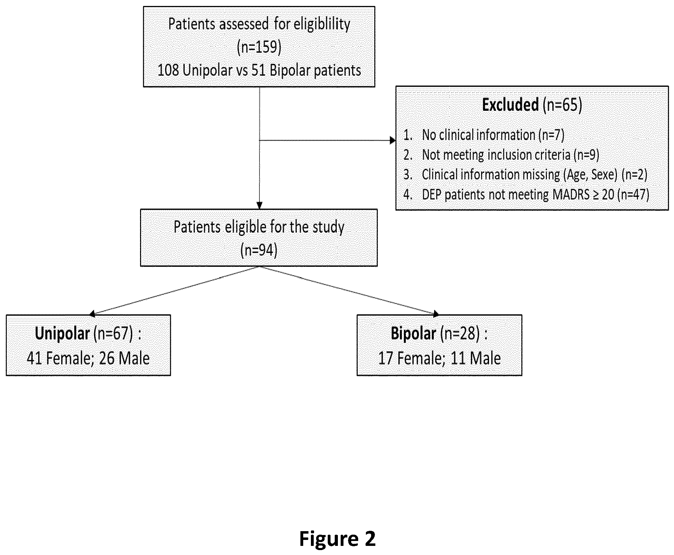

A second cohort of 94 caucasian depressed patients (67 unipolar and 28 bipolar) was recruited among Les Toises - Psychiatry and psychotherapy center of Lausanne, Switzerland.
All participants, aged between 18 or older, understood and signed a written informed consent before entering the study. Diagnosis for unipolar depression and bipolar disorder was made by psychiatrists. Depression severity levels, i.e. mild, moderate and severe were defined by $7 \leq MADRS \leq 19$, $20 \leq MADRS \leq 34$, $MADRS \geq 35$, respectively. Manic symptoms were evaluated by the Young Mania Rating Scale (YMRS). Presence of Bipolar Disorder was assessed by clinician's expertise. All the patients received a treatment classified into these 5 categories: anxiolytics, hypnotics_and_sedatives, antidepressants, antipsychotics, and antiepileptics. The complete flow diagram of the patients through the study is shown in Figure 2 (Figure2: STARD flow diagram of LesToises participants (Study 3))

1.2. Inclusion criteria

**Study 1 & 2**

All subjects must meet the following inclusion criteria:

- Subject aged 18 to 65
- Subject having signed the informed consent
- Able to understand the nature, purpose and methodology of the study
- Able to understand and perform clinical and neuropsychological assessments.
- Subject whose main psychiatric diagnosis is a major depressive episode according to DSM-5 criteria (the existence of psychiatric comorbidities is not a non-inclusion criterion).
- Non pregnant woman.

**Study 3**

All subjects must meet the following inclusion criteria:

- Subject aged 18 or older
- Subject having signed the informed consent
- Able to understand the nature, purpose and methodology of the study
- Able to understand and perform clinical and neuropsychological assessments.
- Subject with harmful substance use will not be considered.
- Subject whose main psychiatric diagnosis is a major depressive episode according to DSM-5 criteria (the existence of psychiatric comorbidities is not a non-inclusion criterion).

We chose to include in both study only patients whose depression severity levels were moderate and severe (MADRS>20 and/or IDSC-30>24), to avoid "borderline" patients, for example, patients with low depression scores or patients for whom one depression scale defined them as depressed while the other defined them as healthy.

1.3. Biological samples

The patients included in both study are subjected to blood samples including a standard work-up (complete blood count) as well as 2 whole blood samples in PAXgene tubesTM to allow the extraction of total RNA from the blood cells and the measurements of RNA editing of targets

**EXAMPLE 2: Methodological approach**

A description of this process analysis is more detailed below and in the publication Salvetat & al, Translational Psychiatry (2022)12:182 [12]

2.1. RNA extraction and qualification from whole blood

Samples were retrieved in PAXgene™ blood RNA tubes, distributed randomly in different sets of extractions and isolated using MagNA Pure 96 instrument (Roche) (LifeScience), according to the manufacturer's protocol. Total RNA concentrations and quality levels were determined with Qubit Fluorometer (Invitrogen) and LabChip (Perkin-Elmer, HT RNA Reagent Kit) instruments, respectively.

2.2. Library preparation for RNA-Sequencing in discovery cohort

For RNA-Seq library we used a TruSeq Stranded Total RNA library kit (Illumina), specifically tailored for blood samples, according to the manufacturer's instructions. Briefly, 300ng total RNAs were depleted in rRNA and globin mRNA (Ribo-Zero Globin), purified and fragmented into 250bp fragments in average. First strand of cDNA was synthetized using Superscript II Reverse Transcriptase (Thermo Fisher Scientific, random primers) and the second strand cDNA using DNA Polymerase I and RNase H (UTP incorporation). DNA fragments were selectively enriched to obtain the library, then normalized, denatured (0.1M NaOH) and sequenced (High-Output, 2 x 75 bp read length) on an Illumina NextSeq 500. Approximately 70 million reads per sample were acquired.

2.3. Targeted Next Generation Sequencing Library preparation and sequencing

Regions of interest for each gene were amplified with validated primers using Q5 Hot Start High Fidelity enzyme (New England Biolabs) according to manufacturer guidelines, on a Peqstar 96x thermocycler. Quality control was ensured: amplicons purity was determined with Nucleic Acid Analyzer (LabChipGx, Perkin Elmer) and PCR product was quantified with fluorescence-based Qubit method (ThermoFisher Scientific). PCR product was purified with magnetic beads (High Prep PCR MAGbio system, Mokascience), and then quantified (Qubit system) to calculate purification yield. After indexing samples (Q5 Hot Start High fidelity PCR enzyme, Nextera XT index kit of Illumina), they were pooled and purified (Magbio PCR cleanup system). Resulting library was denatured (0.1M NaOH), spiked in with PhiX Control V3 (Illumina), loaded onto a sequencing cartridge (Illumina MiSeq Reagent Kit V3 or Illumina NextSeq 500/550 Mid-Output) according to Illumina's guidelines, and sequenced ($1 \times 150$ bp read length) at standard concentrations using ultra-deep sequencing. Approximately 1 million reads per sample were acquired.

2.4. Bioinformatics analysis of A-to-I RNA editome data

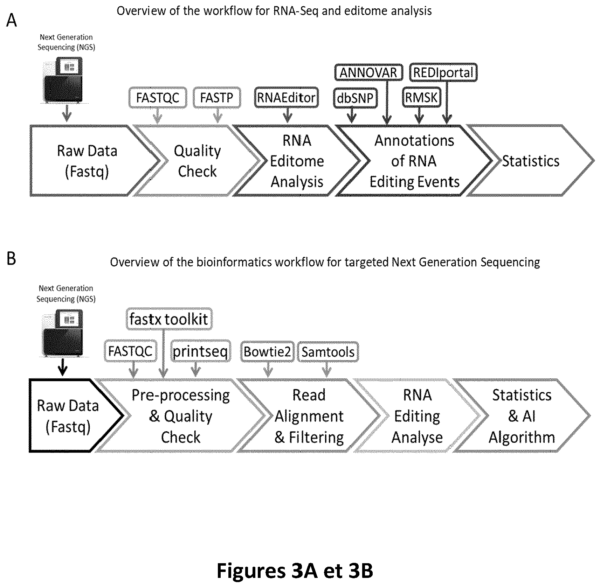

Paired-end reads generated by Illumina NextSeq 500 were demultiplexed using bcl2fastq (version 2.17.1.14, Illumina). Sequencing quality was performed using FastQC (version 0.11.7) and MultiQC software. Identification and quantification of A-to-I editing events were performed using RNAEditor (Version 1.0) with default parameters. RNAEditor accepts FASTQ files as input and implements a workflow fully automated, which comprises a mapping step with BWA using the human genome version (GRCh38 release-83), followed by a step of PCR duplicates removal, a local realignment and a step of base quality score recalibration with Genome Analysis Tool Kit (GATK4). For detection of RNA editing events, Unified Genotyper of GATK4 is used followed by several steps of purifications to reduce the false positives number (known SNPs are excluded, variants in splice junctions are erased, variants in homopolymers are removed) and finally, a step of RNA editing events annotation. We used high confident editing filtering criteria for further analysis (base quality >25, mapping quality>20, mean/median coverage > 30x, min edited reads$\geq$2, editing degree change $\geq$10%, remove editing with 100% editing degree and Wilcoxon's test p-value <0.05). Functional annotation of edited events was then performed with ANNOVAR, RepeatMasker (http://repeatmasker.org) and REDIportal. Alu editing index (AEI) was performed according to the methodology described in Bazak and al. A flowchart of our A-to-I RNA editome analysis is shown in Figure 3A.

2.5. Bioinformatics analysis of targeted sequencing data

The sequencing data were downloaded from the Illumina NextSeq 500. Sequencing quality was performed using FastQC software (version 0.11.7, https://github.com/s-andrews/FastQC/). A minimal sequencing depth of 10 000 reads for each sample was considered for futher analysis. A pretreatment step was performed consisting of removing adapter sequences and filtering of the sequences according to lenght and quality score. Short reads (<100nts) and reads with an average QC<20 were removed. To improve sequence alignment quality, flexible read trimming and filtering tools for Illumina NGS data were used (fastp version ). After performing preprocessing steps, an additional quality control of each cleaned fastq file was carried out prior further analysis. Alignment of the processed reads was performed using bowtie2 (version 2.2.9) with end-to-end sensitive mode. The alignment was done to the reference human genome sequence GRCh38. Non-unique alignments reads, unaligned or reads containing insertion/deletion (INDEL) were removed from downstream analysis by SAMtools software (version 1.7). SAMtools mpileup was used for SVN calling. Finding edited position in the alignment was done by using in-house script's in order to count the number of different nucleotides in each genomic location. For each position, the script computes the percentage of reads that have a 'G' [Number of 'G' reads/ (Number of 'G' reads + Number of 'A' reads)*100]. The genomic location 'A' reference with percentage in 'G' reads > 0.1 are automatically detected by the script and are considered as 'A-to-I edition site'. The last stage is to compute the percentage of all possible isoforms and/or pattern of each transcript. By definition the relative proportion of RNA editing at a given editing 'site' represents the sum of editing modifications measured at this unique genomic coordinate. Conversely, an edited mRNA isoform is a unique molecule that may or may not contain multiple editing modifications on the same transcript. For example for a given transcript, the edited mRNA isoform BC contains an A-to-I modification on both site B and site C within the same transcript. An editing pattern is one combination of editing events occurring at sites of interest in a transcript. By definition, given a list of sites of interest, a transcript has as many editing pattern there are possible combinations. For example for a given transcript, with sites of interest ABC, the analyzed patterns are [A,B,C,AB,BC,AC, ABC]. A relative proportion of at least 0.1% was set as the threshold in order to be included in the analysis. A flowchart of our A-to-I RNA Targeted Next Generation Sequencing pipeline is shown in Figure 3B (Figures 3A and 3B: Overview of our A-I RNA editing analysis. A) overview of workflow for RNA-Seq and editome analysis; B) overview of bioinformatics workflow for Targeted Next Generation Sequencing)

2.6. Statistical analysis of data

All statistics and figures were computed with the "R/Bioconductor" statistical open source software. BMKs (i.e RNA editing sites and edited isoforms/pattern of target genes) values are usually presented as mean $\pm$ standard error of the mean (SEM). In order to guarantee normally distributed data, each biomarker data has been transformed using recipes package v0.2.0 (with step_nzv, step_BoxCox and step_normalize parameters) in each dataset (study2 and study3). A differential analysis was carried out using the most appropriate test between the Mann-Whitney rank-sum test, Student's t-test or Welch's t-test according to normality and sample variance distribution in each cohort. Highly correlated significant variables are remove with find Correlation function from caret package. Significant variables with coefficient variation (CV) upper 30% from repetability and reproducibility analysis have been also removed.

All RNA editing variants selected were combined with age, sex, psychiatric treatment and addiction using extratree algorithm, one of the most recent machine learning algorithm for classification. extraTree method [23], derived from randomForest, requires the use of a training set used to construct the model and a test set to validate it. We have shared our data set: ~60% of the dataset are used for the learning phase and 40% are used for the testing phase. This sharing has been randomized and respect the initial proportion of the various statutes in each sample. We used a grid learning approach for each individual tree, where we stated certain maximum parameter sets (ntree=1000, mtry=(1,20), splitrule="extratrees" and min.node.size=(1,15)). The subtrees are trained with a classical cross-validation. RF results are shown on the test dataset (N=98 samples) from study 2 which has never seen the algorithm and the replication dataset from study3. The implementation was done using the R ranger package (version 0.13.1) and R caret package (version 6.0-90).

**EXAMPLE 3: Results**

3.1 ZNF267 in the discovery cohort (study 1, Editome analysis)

Using our RNA editome pipeline, we identified 40,398 A-to-I edited positions with high degree of confidence in at least one sample (see methods and figure3A). Two major variant types were identified (A-to-G and T-to-C variants), which represent 72.6% of the RNA variants. Most A-to-I editing sites presented moderate editing degree, where 10-30% editing degree accounted for the largest proportion. This RNA edition is found mainly in introns and in 3' untranslated regions (UTRs) as well as in Alu repeat regions and has a homogeneous repartition all over the genome. We then performed a differential analysis to identify sites whose editing could be specifically different between Unipolar and Bipolar disorders, Control vs Depressive and control vs Bipolar Disorder.

To select the genes of interest, we used stringent statistical criteria (coverage>50, p-value <0.05, AUC>0.750, 0.833 > FoldChange > 1.2). ZNF267 is the unique biomarker selected for this diagnostic performance (Table1).

3.2 ZNF267 in the validation cohort (study 2, targeted sequencing analysis)

The analyses of ZN267 RNA editing amplicon on clinical samples (study2) were carried out on our RNA editing measurement platform based on ultra-deep targeted sequencing. Indeed, NGS technologies offer the unique opportunity to study in depth RNA editing events. Our platform measures the modifications of adenosine to inosine (A to I). The biological process is standard with a step of RNA extraction followed by a 2-step PCR sequencing library preparation. For the bioinformatics pipeline, we use our A-I RNA editing targeted NGS pipeline. The bioinformatics pipeline is fully automated and parallelized (Figure 3B).

3.2.1 ZNF267 primers used for the detection of RNA editing sites

Validated primers are used to amplify the region of interest and PCR products are purified and sequenced by NGS (see table2).

3.2.2 Quality control of sequencing data

All quality criteria and requirements are satisfy to apply our algorithm:

- The coverage per biomarker after filtering must be greater than 10,000 reads.
- The percentage of sequences correctly sequenced after filtering (Q25) is greater than 80%.

3.2.3 Differential biomarkers of ZNF267 in cohort2 UNvsBP

In this study the terms "biomarker" refers to an editing site or isoform or pattern of RNA that contains one or more positions differently edited in UN vs BD comparison.

A differential analysis of all detected RNA editing biomarkers was performed on a large cohort of 245 participants for ZNF267 amplicon. 58 biomarkers are found statistically significant with an adjusted p-value <0.05 between depressed unipolar vs depressed bipolar in validation cohort (Table3).

3.2.4 mROC combination of ZNF267 biomarkers in cohort2 UNvsBP

To evaluate the diagnostic performance of ZNF267 significant biomarkers in cohort2, we have used mROC approach, a dedicated program to identify the linear combination, which maximizes the AUC (Area Under the Curve) ROC. The result of top 5 best combination obtained is shown in table 4.

3.3 ZNF267 associated with others biomarkers of interest (study 2 and 3, targeted sequencing analysis)

In addition to ZNF267 biomarkers, we have selected 7 targets of interest for the diagnosis of depressed unipolar versus depressed bipolar on the basis of our previous results [salvetat & al, transl psych 2020 and WO2021089866A1]. These targets are IFNAR1, GAB2, IL17RA, LYN, MDM2, PRKCB and PTPRC. The same A-I RNA editing targeted NGS pipeline as for ZNF267 was applied.

To combine all A-to-I RNA editing biomarkers, we have used machine learning approaches (extraTrees, R package caret and ranger) to develop clinical decision algorithms.

The top5 best algorithms performances using 8 targets are represented in table5.

With the same approach, the best algorithms performances using 7 targets without ZNF267 are represented in Table 6. As we can see, the best performances are obtained with ZNF267 (see Table 5).

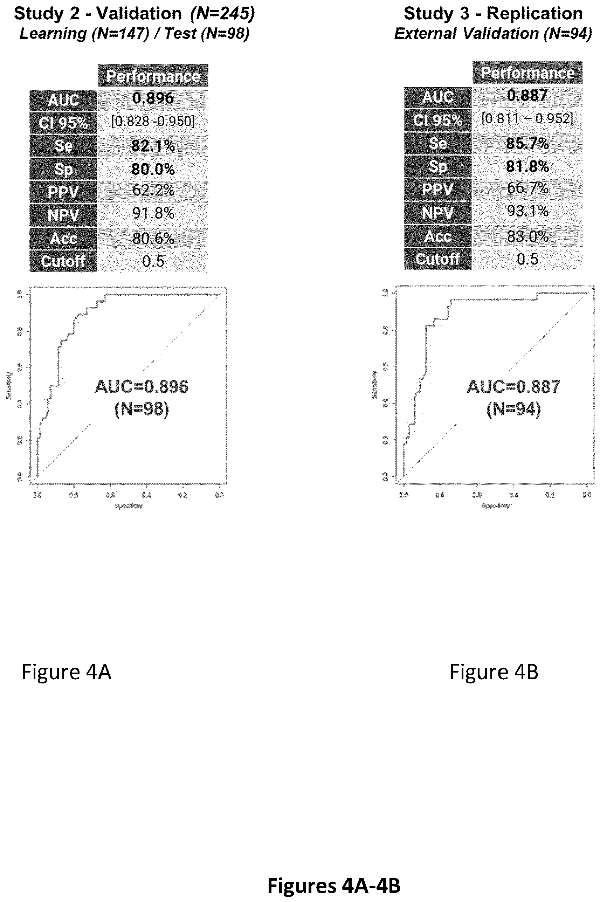

For example 1, extratree algorithm model #154 gave an AUC ROC curve of 0.896 (CI 95%: [0.835 - 0.956]), with a sensitivity of 82.1% and a specificity of 80.0% (see Figure 4), allowing a clear separation of unipolar from bipolar patients on study2 test dataset (internal validation). The same model (#154) gave an AUC ROC curve of 0.887 (CI 95%: [0.811 - 0.952]), with a sensitivity of 85.7% and a specificity of 81.8% (see Figure 4) on study3 replication dataset (external validation), allowing to confirm, on independent cohort, the diagnostic performance of study 2 (Figure 4: Receiver operating characteristic curve and diagnostic performances for bipolar depression on both study for model #154).

Plotted are the probabilities for the correct response of the tested sets using case specific trained random forest models (extraTree model #154). The test dataset on study 2 (internal validation) contains 70 patients from the unipolar group and 28 from bipolar patients. Replication dataset on study3 (external validation) contains 67 patients from the unipolar group and 28 from bipolar patients. Number of Target used: 8 (GAB2, IFNAR1, LYN, MDM2, PTPRC, IL17RA, ZNF267 and PRKCB ) and number of RNA editing variant used: 22 (extraTree model #154).

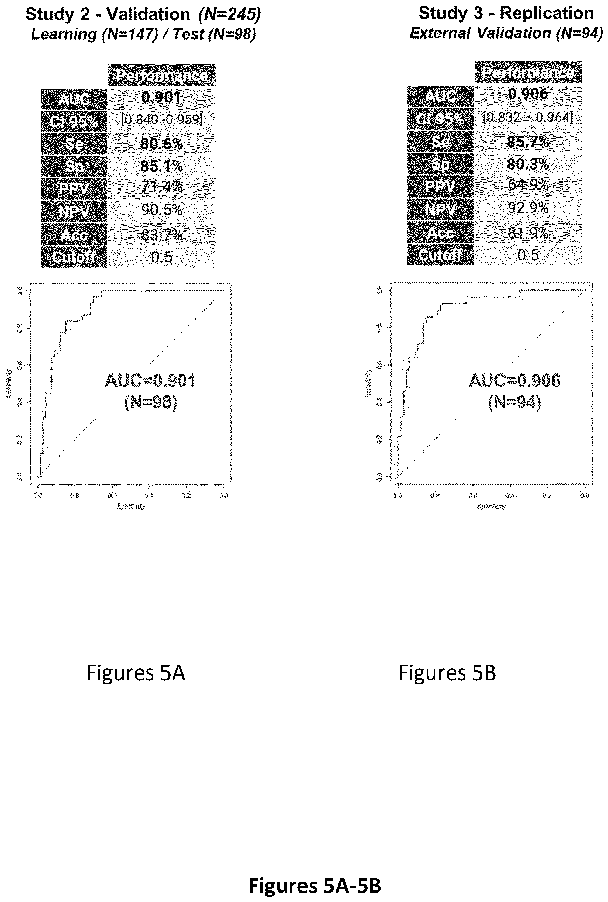

For example 2, extratree algorithm model #284 gave an AUC ROC curve of 0.901 (CI 95%: [0.840 - 0.959]), with a sensitivity of 80.6% and a specificity of 85.1% (see Figure 5), allowing a clear separation of unipolar from bipolar patients on study2 test (internal validation)dataset. The same model (#284) gave an AUC ROC curve of 0.906 (CI 95%: [0.832 - 0.964]), with a sensitivity of 85.7% and a specificity of 80.3% (see Figure 5) on study3 replication (external validation) dataset, allowing to confirm, on independent cohort, the diagnostic performance of study 2.

Figure 5: Receiver operating characteristic curve and diagnostic performances for bipolar depression on both study for model #284.

Plotted are the probabilities for the correct response of the tested sets using case specific trained random forest models (extraTree model #284). The test dataset on study 2 (internal validation) contains 70 patients from the unipolar group and 28 from bipolar patients. Replication dataset on study3 (external validation) contains 67 patients from the unipolar group and 28 from bipolar patients. Number of Target used: 8 (GAB2, IFNAR1, LYN, MDM2, PTPRC, IL17RA, ZNF267 and PRKCB ) and number of RNA editing variant used: 22 (extraTree model #284).

**Bibliography :**

1. Judd LL, Akiskal HS, Schettler PJ, Endicott J, Maser J, Solomon DA, et al. The longterm natural history of the weekly symptomatic status of bipolar I disorder. Arch Gen Psychiatry. 2002;59:530-7.

2. Jung Y, Goldman D. Role of RNA modifications in brain and behavior. Genes Brain Behav. 2018;17:e12444.

3. Shelton RC. The molecular neurobiology of depression. Psychiatr Clin North Am. 2007;30:1-11.

4. Lee SY, Lu RB, Wang LJ, Chang CH, Lu T, Wang TY, et al. Serum miRNA as a possible biomarker in the diagnosis of bipolar II disorder. Sci Rep. 2020;10:1131.

5. Giacopuzzi E, Gennarelli M, Sacco C, Filippini A, Mingardi J, Magri C, et al. Genome-wide analysis of consistently RNA edited sites in human blood reveals interactions with mRNA processing genes and suggests correlations with cell types and biological variables. BMC Genomics. 2018;19:963.

6. Yang W, Wang Q, Kanes SJ, Murray JM, Nishikura K. Altered RNA editing of serotonin 5-HT2C receptor induced by interferon: implications for depression associated with cytokine therapy. Brain Res Mol Brain Res. 2004;124:70-8.

7. Lyddon R, Dwork AJ, Keddache M, Siever LJ, Dracheva S. Serotonin 2c receptor RNA editing in major depression and suicide. World J Biol Psychiatry. 2013;14:590-601.

8. Weissmann D, van der Laan S, Underwood MD, Salvetat N, Cavarec L, Vincent L, et al. Region-specific alterations of A-to-I RNA editing of serotonin 2c receptor in the cortex of suicides with major depression. Transl Psychiatry. 2016;6:e878.

9. Chimienti F, Cavarec L, Vincent L, Salvetat N, Arango V, Underwood MD, et al. Brain region-specific alterations of RNA editing in PDE8A mRNA in suicide decedents. Transl Psychiatry. 2019;9:91.

10. Salvetat N, Van der Laan S, Vire B, Chimienti F, Cleophax S, Bronowicki JP, et al. RNA editing blood biomarkers for predicting mood alterations in HCV patients. J Neurovirol. 2019;25:825-36.

11. Salvetat N, Chimienti F, Cayzac C, Dubuc B, Checa-Robles F, Dupre P, et al. Phosphodiesterase 8A to discriminate in blood samples depressed patients and suicide attempters from healthy controls based on A-to-I RNA editing modifications. Transl Psychiatry. 2021;11:255.

12. Salvetat N, Checa-Robles FJ, Patel V, Cayzac C, Dubuc B, Chimienti F, Abraham JD, Dupré P, Vetter D, Méreuze S, Lang JP, Kupfer DJ, Courtet P, Weissmann D. A game changer for bipolar disorder diagnosis using RNA editing-based biomarkers. Transl Psychiatry. 2022 May 4;12(1):182.

13. Bueno-Notivol J, Gracia-García P, Olaya B, Lasheras I, López-Antón R, Santabárbara J. Prevalence of depression during the COVID-19 outbreak: a metaanalysis of community-based studies. Int J Clin Health Psychol. 2021;21:100196.

14. Wu T, Jia X, Shi H, Niu J, Yin X, Xie J, et al. Prevalence of mental health problems during the COVID-19 pandemic: A systematic review and meta-analysis. J Affect Disord. 2021;281:91-98.

15. Schnabl B, Hu K, Muhlbauer M, Hellerbrand C, Stefanovic B, Brenner DA, et al. (2005): Zinc finger protein 267 is up-regulated during the activation process of human hepatic stellate cells and functions as a negative transcriptional regulator of MMP-10. Biochemical and biophysical research communications. 335:87-96.

16. Schnabl B, Valletta D, Kirovski G, Hellerbrand C (2011): Zinc finger protein 267 is up-regulated in hepatocellular carcinoma and promotes tumor cell proliferation and migration. Experimental and molecular pathology. 91:695-701.

17. Schnabl B, Czech B, Valletta D, Weiss TS, Kirovski G, Hellerbrand C (2011): Increased expression of zinc finger protein 267 in non-alcoholic fatty liver disease. International journal of clinical and experimental pathology. 4:661-666.

18. Lu L, Chen XM, Tao HM, et al. (2015) Regulation of the expression of zinc finger protein genes by microRNAs enriched within acute lymphoblastic leukemia-derived microvesicles. Genet Mol Res. 14(4):11884-1189

19. Cheishvili D, Parashar S, Mahmood N, Arakelian A, Kremer R, Goltzman D, et al. (2018): Identification of an Epigenetic Signature of Osteoporosis in Blood DNA of Postmenopausal Women. Journal of bone and mineral research : the official journal of the American Society for Bone and Mineral Research. 33:1980-1989.

20. Patel S, Howard D, Chowdhury N, et al. (2021) Characterization of human genes modulated by por-phyromonas gingivalis highlights the ribosome, hypothalamus, and cholinergic neurons. Front Immunol. 12:646259.

21. Fehlbaum-Beurdeley P, Sol O, Désiré L, Touchon J, Dantoine T, Vercelletto M, et al. (2012) Validation of AclarusDxTM, A Blood-Based Transcriptomic Signature for the Diagnosis of Alzheimer's Disease. J Alzheimers Dis. 32:169-81.

22. Nguyen HD, Jo WH, Hoang NHM, Kim MS. Curcumin-Attenuated TREM-1/DAP12/NLRP3/Caspase-1/IL1B, TLR4/NF-$\kappa$B Pathways, and Tau Hyperphosphorylation Induced by 1,2-Diacetyl Benzene: An in Vitro and in Silico Study. Neurotox Res. 2022 Oct;40(5):1272-1291.

23. Pierre Geurts · Damien Ernst · Louis Wehenkel. Extremely randomized trees. Mach Learn (2006) 63: 3-42 DOI 10.1007/s10994-006-6226-1

**Table 1:** Differentially A-To-I edited sites of ZNF267 gene between Healthy controls vs depressed patients, Healthy controls vs depressed bipolar patients and depressed unipolar vs depressed bipolar patients.

| Ensembl ID | GeneName | RegionChr | Position (GRCh38) | Coverage | foldChange | p valueAUC | comparison | |
|---|---|---|---|---|---|---|---|---|
| ENSG00000185947 | ZNF267 | intron 16 | 31892121 | 63 | 0.79 | 0.00602 | 0.840 | UN-DEP vs BP-DEP |
| | | | | 62 | 0.70 | 0.00001 | 0.910 | CtrlvsBP-DEP |
| | | | | 63 | 0.81 | 0.00306 | 0.750 | CtrlvsDEP |

**Table 2:** ZNF267 validated primers for the detection of RNA editing sites

| PRIMER NAME | Gene Name | PCR product Size (bp) | Primer Sequence (5'->3') | Primer length (bp) | SEQ ID NO. |
|---|---|---|---|---|---|
| ZNF267 MPx_F2 | ZNF267 | 151 | GGCTGAGGTGGTCTTGGATG | 20 | 1 |
| ZNF267 MPx_R1 | | | CCTCGCCTTCCACTGTGATT | 20 | 2 |

**Table 3:** Differentially A-To-I edited biomarkers of ZNF267 amplicon between depressed unipolar vs depressed bipolar in internal validation cohort (study2)

| NB | Biomarkers | Median_ Editing(%) | Mean_ Editing(%) | foldMedian | foldChange | pValue | pValue_ FDR |
|---|---|---|---|---|---|---|---|
| 1 | ZNF267_pat_ ACG | 3.77 | 4.08 | 1.34 | 1.33 | 0.0001 | 0.0212 |
| 2 | ZNF267_pat_ ACGL | 3.91 | 4.26 | 1.31 | 1.35 | 0.0004 | 0.0212 |
| 3 | ZNF267_pat_ ACGK | 2.07 | 2.44 | 1.34 | 1.39 | 0.0004 | 0.0212 |
| 4 | ZNF267_pat_CG | 4.50 | 4.75 | 1.18 | 1.20 | 0.0005 | 0.0212 |
| 5 | ZNF267_pat_AH | 3.62 | 3.96 | 1.23 | 1.19 | 0.0005 | 0.0233 |
| 6 | ZNF267_pat_ ACGW | 3.02 | 3.37 | 1.22 | 1.35 | 0.0007 | 0.0212 |
| 7 | ZNF267_pat_ ACDG | 2.89 | 3.26 | 1.17 | 1.33 | 0.0009 | 0.0212 |
| 8 | ZNF267_pat_ ABH | 1.55 | 1.77 | 1.23 | 1.36 | 0.0009 | 0.0212 |
| 9 | ZNF267_pat_AHI | 3.57 | 3.80 | 1.19 | 1.21 | 0.0009 | 0.0233 |
| 10 | ZNF267_pat_ ACGKW | 1.84 | 2.12 | 1.36 | 1.48 | 0.0010 | 0.0212 |
| 11 | ZNF267_pat_AHL | 3.91 | 4.18 | 1.13 | 1.23 | 0.0011 | 0.0212 |
| 12 | ZNF267_pat_ ACGLW | 2.91 | 3.35 | 1.27 | 1.36 | 0.0012 | 0.0212 |
| 13 | ZNF267_pat_ AHIW | 1.21 | 1.34 | 1.28 | 1.41 | 0.0014 | 0.0213 |
| 14 | ZNF267_pat_ CGKW | 1.75 | 2.07 | 1.30 | 1.42 | 0.0016 | 0.0214 |
| 15 | ZNF267_pat_ CDG | 2.98 | 3.27 | 1.23 | 1.26 | 0.0016 | 0.0214 |
| 16 | ZNF267_pat_CH | 1.77 | 1.96 | 1.33 | 1.29 | 0.0018 | 0.0219 |
| 17 | ZNF267_site_ 31892117_I | 21.41 | 21.47 | 1.04 | 1.04 | 0.0019 | 0.0245 |
| 18 | ZNF267_pat_ ABCGL | 1.73 | 1.97 | 1.34 | 1.37 | 0.0021 | 0.0230 |
| 19 | ZNF267_pat_ AHILW | 1.01 | 1.30 | 1.64 | 1.47 | 0.0021 | 0.0230 |
| 20 | ZNF267_pat_ ACGKL | 2.17 | 2.54 | 1.27 | 1.39 | 0.0022 | 0.0235 |
| 21 | ZNF267_pat_KM | 4.99 | 5.09 | 1.10 | 1.15 | 0.0023 | 0.0238 |
| 22 | ZNF267_pat_AIW | 6.24 | 6.44 | 1.20 | 1.17 | 0.0023 | 0.0238 |
| 23 | ZNF267_pat_ ACDGW | 2.67 | 3.11 | 1.33 | 1.32 | 0.0027 | 0.0252 |

(continued)

| NB | Biomarkers | Median_ Editing(%) | Mean_ Editing(%) | foldMedian | foldChange | pValue | pValue_ FDR |
|---|---|---|---|---|---|---|---|
| 24 | ZNF267_pat_ CGL | 4.96 | 5.14 | 1.15 | 1.21 | 0.0030 | 0.0262 |
| 25 | ZNF267_pat_ CGK | 2.63 | 2.90 | 1.24 | 1.28 | 0.0031 | 0.0265 |
| 26 | ZNF267_pat_AI | 12.92 | 13.01 | 1.09 | 1.10 | 0.0032 | 0.0269 |
| 27 | ZNF267_pat_ BDILW | 1.12 | 1.53 | 1.59 | 1.40 | 0.0032 | 0.0293 |
| 28 | ZNF267_pat_ CGW | 2.87 | 3.17 | 1.29 | 1.25 | 0.0034 | 0.0275 |
| 29 | ZNF267_pat_ ACGKLW | 1.78 | 2.20 | 1.35 | 1.47 | 0.0035 | 0.0279 |
| 30 | ZNF267_pat_ ABCG | 1.62 | 1.87 | 1.45 | 1.32 | 0.0038 | 0.0290 |
| 31 | ZNF267_pat_ACI | 4.38 | 4.70 | 1.19 | 1.20 | 0.0045 | 0.0309 |
| 32 | ZNF267_pat_CI | 5.50 | 5.68 | 1.10 | 1.14 | 0.0046 | 0.0312 |
| 33 | ZNF267_pat_ AILW | 5.50 | 5.83 | 1.16 | 1.19 | 0.0049 | 0.0321 |
| 34 | ZNF267_pat_ CGKLW | 1.85 | 2.24 | 1.41 | 1.42 | 0.0052 | 0.0329 |
| 35 | ZNF267_pat_ ABCL | 2.82 | 3.01 | 1.27 | 1.23 | 0.0052 | 0.0351 |
| 36 | ZNF267_pat_ BCDILW | 1.08 | 1.50 | 1.84 | 1.49 | 0.0053 | 0.0332 |
| 37 | ZNF267_pat_ KMW | 2.20 | 2.47 | 1.20 | 1.27 | 0.0053 | 0.0332 |
| 38 | ZNF267_pat_ ABCDG | 1.33 | 1.66 | 1.25 | 1.40 | 0.0055 | 0.0339 |
| 39 | ZNF267_pat_ ABCDILW | 1.05 | 1.50 | 2.13 | 1.52 | 0.0056 | 0.0342 |
| 40 | ZNF267_pat_AC | 8.69 | 8.97 | 1.05 | 1.11 | 0.0059 | 0.0352 |
| 41 | ZNF267_site_ 31892116_H | 5.95 | 5.99 | 1.06 | 1.08 | 0.0063 | 0.0375 |
| 42 | ZNF267_pat CGLW | 2.82 | 3.34 | 1.21 | 1.27 | 0.0064 | 0.0364 |
| 43 | ZNF267_pat_CHL | 1.69 | 2.05 | 1.34 | 1.30 | 0.0064 | 0.0364 |
| 44 | ZNF267_pat_ ABILW | 1.81 | 2.21 | 1.47 | 1.33 | 0.0071 | 0.0383 |
| 45 | ZNF267_pat_ AHIL | 3.27 | 3.50 | 1.32 | 1.23 | 0.0073 | 0.0388 |
| 46 | ZNF267_pat_ CDGW | 2.45 | 2.79 | 1.26 | 1.27 | 0.0079 | 0.0407 |
| 47 | ZNF267_pat_ ACH | 1.35 | 1.67 | 1.28 | 1.33 | 0.0081 | 0.0413 |
| 48 | ZNF267_pat_ BCDIW | 1.14 | 1.54 | 1.55 | 1.37 | 0.0081 | 0.0423 |
| 49 | ZNF267_pat_ ACHL | 1.36 | 1.71 | 1.35 | 1.39 | 0.0084 | 0.0423 |
| 50 | ZNF267_pat_ ACDGL | 2.77 | 3.24 | 1.32 | 1.26 | 0.0086 | 0.0430 |
| 51 | ZNF267_pat_ ABHL | 1.61 | 1.76 | 1.26 | 1.33 | 0.0087 | 0.0433 |

(continued)

| NB | Biomarkers | Median_ Editing(%) | Mean_ Editing(%) | foldMedian | foldChange | pValue | pValue_ FDR |
|---|---|---|---|---|---|---|---|
| 52 | ZNF267_pat_ ACGI | 1.86 | 2.19 | 1.27 | 1.32 | 0.0089 | 0.0445 |
| 53 | ZNF267_pat_ ABCILW | 1.15 | 1.46 | 1.70 | 1.42 | 0.0094 | 0.0454 |
| 54 | ZNF267_pat_ ADGW | 2.83 | 3.23 | 1.18 | 1.23 | 0.0095 | 0.0462 |
| 55 | ZNF267_pat_ KLM | 4.71 | 4.92 | 1.20 | 1.16 | 0.0096 | 0.0461 |
| 56 | ZNF267_pat_ACL | 8.28 | 8.59 | 1.10 | 1.12 | 0.0100 | 0.0472 |
| 57 | ZNF267_pat_CGI | 2.77 | 3.07 | 1.21 | 1.21 | 0.0102 | 0.0477 |
| 58 | ZNF267_pat_ AHLW | 1.82 | 2.04 | 1.27 | 1.31 | 0.0107 | 0.0493 |

**Table 4:** diagnostic performance of ZNF267 editing biomarkers combinations between depressed unipolar vs depressed biopolar in internal validation cohort (study2)

| Num | ZNF267 biomarkers combinations | AUC ROC | AUC CI 95% | cut-off | Sp (%) | Se (%) | VPP (%) | VPN (%) |
|---|---|---|---|---|---|---|---|---|
| 1 | ZNF267_C57_N2 | 0.803 | [ 0.748; 0.858 ] | 1.863 | 72.4 | 72.5 | 60.6 | 81.9 |
| 2 | ZNF267_C57_N57 | 0.801 | [ 0.745 ; 0.856 ] | 1.861 | 81.4 | 64.8 | 67.1 | 79.9 |
| 3 | ZNF267_C57_N43 | 0.800 | [ 0.744 ; 0.855 ] | 1.917 | 82.1 | 63.7 | 67.4 | 79.5 |
| 4 | ZNF267_C57_N5 | 0.800 | [ 0.745 ; 0.856 ] | 1.775 | 73.1 | 71.4 | 60.8 | 81.4 |
| 5 | ZNF267_C57_N55 | 0.799 | [ 0.744 ; 0.855 ] | 2.059 | 70.5 | 74.7 | 59.7 | 82.7 |

**Table 5:** Top5 best extratree model using ZNF267 to diagnose depressed unipolar vs depressed bipolar

| Num | Learning / Test (60% / 40%) | Validation | Method | #BMKs | #Targets | AUC Test | Se Test | Sp Test | Acc Test | AUC Valid | Se valid | Sp valid | Acc val | Model # |
|---|---|---|---|---|---|---|---|---|---|---|---|---|---|---|
| 1 | Study2 - CHU Montpellier (N=245) | Study3 - LesToises (N=98) | extra tree | 21 | 8 | **0.931** | 88.2 | 83.3 | 85.4 | **0.878** | 82.1 | 81.8 | 81.9 | 153 |
| 2 | Study2 - CHU Montpellier (N=245) | Study3 - LesToises (N=98) | extra tree | 22 | 8 | **0.922** | 82.7 | 84 | 83.7 | **0.892** | 92.8 | 81.8 | 85.1 | 90 |
| 3 | Study2 - CHU Montpellier (N=245) | Study3 - LesToises (N=98) | extra tree | 22 | 8 | **0.901** | 80.6 | 85 | 83.7 | **0.906** | 85.7 | 80.3 | 81.9 | 284 |
| 4 | Study2 - CHU Montpellier (N=245) | Study3 - LesToises (N=98) | extra tree | 22 | 8 | **0.896** | 82.1 | 80 | 80.6 | **0.887** | 85.7 | 81.8 | 83.0 | 154 |
| 5 | Study2 - CHU Montpellier (N=245) | Study3 - LesToises (N=98) | extra tree | 21 | 8 | **0.872** | 82.1 | 92.8 | 89.8 | **0.870** | 82.1 | 80.3 | 80.9 | 302 |

**Table 6:** Example of extratree model using 6 targets of interest without ZNF267 to diagnose depressed unipolar vs depressed bipolar

| Num | Learning / Test (60%, / 40%) | Validation | Method | #BMKs | #Targets | AUC Test | Se Test | Sp Test | Acc Test | AUC valid | Se valid | Sp valid | Acc valid | Model # |
|---|---|---|---|---|---|---|---|---|---|---|---|---|---|---|
| 1 | Study2-CHU Montpellier (N=245) | Study3 - Les Toises (N=98) | extratree | 20 | 6* | 0,86 | 83,8 | 80,3 | 81,6 | 0,8 | 71,4 | 78,8 | 76,6 | 468 |
| 2 | Study2-CHU Montpellier (N=245) | Study3 - Les Toises (N=98) | extratree | 20 | 6* | 0,857 | 76,3 | 83,3 | 80,6 | 0,844 | 85,7 | 80,3 | 81,9 | 390 |
| 3 | Study2-CHU Montpellier (N=245) | Study3 - Les Toises (N=98) | extratree | 20 | 6* | 0,81 | 82,4 | 76,6 | 78,6 | 0,801 | 89,3 | 62,1 | 70,2 | 447 |
| * Target list: GAB2, IFNAR1, LYN, MDM2, PTPRC, and PRKCB | | | | | | | | | | | | | | |

**Table 7: Primers used for the detection of MDM2, PRKCB, IFNAR1, LYN, GAB2, CAMK1D and KCNJ15 RNA editing sites**

| PRIMER NAME | Gene Name | PCR product Size (bp) | Primer Sequence (5'->3') | Primer length (bp) | SEQ ID NO. |
|---|---|---|---|---|---|
| IFNAR1 FORWARD_1 | IFNAR1 | 259 | ATACGGCAAGCTCTTAACTATT | 23 | 3 |
| IFNAR1 REVERSE_1 | | | AACCTCCACCTCCTGTTTTC | 20 | 4 |
| LYN MPx_F1 | LYN | 193 | TTTAAAAGCTTCTCCAGTCAGC | 22 | 5 |
| LYN MPx_R1 | | | TCCTGAGTAGCTGGGACCAT | 20 | 6 |
| PRKCB_MPx_F1 | PRKCB | 176 | CAGCACATTCTGTTGCCTTC | 20 | 7 |
| PRKCB_MPx_R2 | | | GTGACTTTGCCCCTCATTTG | 20 | 8 |
| MDM2_F5 MPx-Rev | MDM2 | 302 | AAGCCCATCCTCGAACTACAG | 21 | 9 |
| MDM2_R5 MPx-Rev | | | TTGCTCTGTCACCTGGACTG | 20 | 10 |
| GAB2 MPx_F1 | GAB2 | 154 | GAACCAGGCTAGGTGCAATG | 20 | 11 |
| GAB2 MPx_R2 | | | CCATGCCCTGGTAATTTTTG | 20 | 12 |
| IL17RA_Forward1 | IL17RA | 172 | GGGATGTAAACGCTGAATGG | 20 | 13 |
| IL17RA_Reverse1 | | | ATGTGCCACCATGACTGACT | 20 | 14 |
| PTPRC_F4 | PTPRC | 226 | AGGCTTCAAACTGTCTTGT | 21 | 15 |
| PTPRC_R4 | | | CTCACTGCAACCTCCGCC | 19 | 16 |

**Claims**

1. An in vitro method for analyzing the expression of biomarker RNA molecules, comprising the steps of:

A-isolating RNA from a blood sample obtained from a subject and determining the expression of at least one biomarker RNA molecule in the isolated RNA and providing an expression profile based on the results, wherein said biomarker is the relative proportion of RNA editing at at least a given editing site and/or isoform and/or pattern or a combination of given sites and/or isoforms and/or patterns, which can be edited on ZNF267 gene. B-from the same isolated RNA, determining the expression of at least another biomarker RNA molecule in the isolated RNA and providing an expression profile based on the results, wherein said biomarker is the relative proportion of RNA editing at at least a given editing site and/or isoform and/or pattern or a combination of given sites and/or isoforms and/or patterns, which can be edited on another A to I editing RNA gene; and C-using the expression profiles determined in step A and in B for a combined analysis of the results.

2. The method according to claim 1, wherein in step B, said another A to I editing RNA gene is selected from the group of A to I editing RNA genes consisting of GAB2, IFNAR1, LYN, MDM2, PTPRC, IL 17RA and PRKCB A to I editing RNA genes.

3. The method according to claim 1 or 2, wherein in step A, said blood sample is obtained from a patient during depression phase, preferably during moderate or severe depression phase.

4. A method for in vitro differential diagnosis of bipolar disorder versus unipolar disorder in patient during moderate or severe depression phases, the method comprising the determination in a sample of the patient of the relative proportion of RNA editing at at least a given editing site and/or isoform and/or pattern or a combination of given sites and/or isoforms and/or patterns, which can be edited on ZNF267 gene.

5.  The method of claim 4, further comprised the determination preferably in the same sample of the relative proportion of RNA editing at at least a given editing site and/or isoform and/or pattern or a combination of given sites and/or isoforms and/or patterns, which can be edited on another one A to I editing RNA gene selected from the group comprising the GAB2, IFNAR1, LYN, MDM2, PTPRC, IL 17RA and PRKCB A to I editing RNA biomarkers, more preferably on at least 2, 3, 4, 5 or 6 of these genes.

6.  The method of claim 4 or 5, further comprised the determination of the relative proportion of RNA editing at at least a given editing site and/or isoform and/or pattern, or a combination of given sites and/or isoforms and/or patterns, which can be edited on the GAB2, IFNAR1, LYN, MDM2, PTPRC, IL 17RA and PRKCB A to I editing RNA genes.

7.  The method of one of claims 1 to 6 wherein the PCR products of the gene region of interest of the selected A to I editing RNA gene(s) are sequenced by NGS.

8.  The method of one of claims 1 to 7, wherein:

    a) the pair of primers used for obtaining the ZNF267 gene PCR products of interest are:

    | | | |
    |---|---|---|
    | ZNF267 MPx_F2 | GGCTGAGGTGGTCTTGGATG | (SEQ ID No.1) |
    | ZNF267 MPx_R1 | CCTCGCCTTCCACTGTGATT | (SEQ ID No.2); |

    And
    b) the pair of primers (used for obtaining the IFNAR1, LYN, PRKCB, MDM2, GAB2, IL17RA and PTPRC gene PCR products of interest are respectively listed in Table 7 (SEQ ID No.3 to 16).

9.  The method for *in vitro* differential diagnosis of bipolar disorder versus unipolar disorder in patient during moderate or severe depression phases of one of claims 4 to 8 said method comprising the step of:

    a) determining in a sample of the patient to be tested the relative proportion of RNA editing at at least a given editing site and/or isoform and/or pattern, or combination of given sites and/or isoforms and/or patterns, which can be edited on the ZNF267 mRNA gene, and optionally which can be edited on at least another one of the GAB2, IFNAR1, LYN, MDM2, PTPRC, IL17RA and PRKCB A to I editing RNA gene, preferably 2, 3, 4, 5, 6 or 7 of these genes;.
    b) determining a value resulting from the proportion(s) obtained in step a);
    c) classifying said patient as being bipolar or unipolar after comparing said resulting value obtained in step b) to a control value obtained for bipolar and/or unipolar patients, , wherein said control values were determined in a manner comparable to that of the resulting value obtained in step b).

10. The method of claim 9, wherein in step c) said resulting value obtained for the patient to be tested is compared to a threshold associated to the disorder which is desired to be identified (for example, but non-limited to a cut-off or a probability), classifying said patient as being bipolar or unipolar patient according to the chosen threshold if said resulting value is equal to , or greater or smaller than said threshold.

11. The method of one of claims 1 to 10, wherein the sample of the patient is a biological sample, preferred is a blood, serum, urine, saliva, tear fluid or plasma sample, more preferably a blood sample, blood serum, even more preferably the sample of the patient is a blood sample, even more preferably a blood serum sample.

12. A method for determining the effectiveness of a therapy in a subject or for predicting or monitoring therapy response in a patient is provided, comprising:

    I-analyzing the expression of biomarker RNA molecules according to the method of claims 1 to 3 before and/or during and/or after treatment of the patient;
    and
    II-Using the expression profiles determined in step C for each of the analyses for a combined analysis of the results.

13. A method for monitoring treatment for bipolar disorder or unipolar disorder in a human patient during moderate or severe depression phases, from a biological sample of said patient, said method comprising:

A) differential diagnosing of bipolar versus unipolar disorder in said human by the method of anyone of claims 9 and 10before the beginning of the treatment which is desired to be monitored.
B) repeating steps (a) to c) of the method after a period of time during which said patient receives the desired treatment for said diagnosed bipolar or unipolar disorder, in order to obtain a post-treatment result value;
C) comparing the post-treatment result value from step (c) to the result value obtained before treatment.

14. A method for determining whether a patient during depression phases will be a responder to a bipolar or an unipolar disorder treatment allowing the patient to be in a euthymic state (state of normal mood) from a biological sample, said method comprising the step of:

A) differential diagnosing of bipolar versus unipolar disorder in said human by the method of anyone of claims 9 to 10 before the beginning of the treatment which is desired to be monitored.
B) repeating steps (a) to c) of the method of the present invention, after a period of time during which said patient receives the desired treatment for said diagnosed bipolar or unipolar disorder, to obtain a post-treatment result value;
C) comparing the post-treatment result value from step (c) to:

- the result value obtained before treatment, and
- to the result value known or obtained for control bipolar or unipolar disorder patients being in an euthymic state, and

classifying said patient as being responder to the treatment if the post-treatment result value obtained in step B) is closer of said euthymic state result value that said result value obtained before treatment.

15. A kit for differential diagnosing of bipolar versus unipolar disorder in a human patient during moderate or severe depression phases, said kit comprising:

1) - optionally, instructions to apply the method for differential diagnosing of bipolar versus unipolar disorder in a human patient during depression phases, in order to obtain a resulting value the analysis of which determining whether said depressed patient presents a bipolar or unipolar disorder; and
2)

a) the pair of primers having the sequence SEQ ID Nos. 1 and 2, and
b) at least 1 pair, preferably 2, 3, 4, 5, 6 or the 7 pairs of primers selected from the group of pairs of primers having the sequences SEQ ID Nos.3 to 16, more preferably the 7 pairs of primers having the sequences SEQ ID No.3 to 16.

16. A kit for differential diagnosing of bipolar versus unipolar disorder in a human patient during moderate or severe depression phases, said kit comprising:

1) - optionally, instructions to apply the method for differential diagnosing of bipolar versus unipolar disorder in a human patient during depression phases, in order to obtain a resulting value the analysis of which determining whether said depressed patient presents a bipolar or unipolar disorder; and
2)

a) a pair of primers allowing to obtain an amplicon including or identical to the amplicon obtainable by the pair of primers having the sequences SEQ ID Nos. 1 and 2; and
b) at least 1 pair, preferably 2, 3, 4, 5, 6 or 7 pairs of primers allowing to obtain amplicon(s) including or identical to the amplicon(s) obtainable by the pairs of primers selecting from the group of pair of primers having the sequences SEQ ID Nos.3 to16, preferably 7 pairs of primers allowing to obtain 7 amplicon(s) including or identical to the 7 amplicons obtainable by the 7 pairs of primers having the sequences SEQ ID Nos.3 to16.

**Figure 1**

Figure 2

A — Overview of the workflow for RNA-Seq and editome analysis

B — Overview of the bioinformatics workflow for targeted Next Generation Sequencing

**Figures 3A et 3B**

**Study 2 - Validation** *(N=245)*
*Learning (N=147) / Test (N=98)*

| | Performance |
|---|---|
| AUC | **0.896** |
| CI 95% | [0.828 -0.950] |
| Se | **82.1%** |
| Sp | **80.0%** |
| PPV | 62.2% |
| NPV | 91.8% |
| Acc | 80.6% |
| Cutoff | 0.5 |

**Study 3 - Replication**
*External Validation (N=94)*

| | Performance |
|---|---|
| AUC | **0.887** |
| CI 95% | [0.811 – 0.952] |
| Se | **85.7%** |
| Sp | **81.8%** |
| PPV | 66.7% |
| NPV | 93.1% |
| Acc | 83.0% |
| Cutoff | 0.5 |

AUC=0.896
(N=98)

AUC=0.887
(N=94)

Figure 4A                Figure 4B

**Figures 4A-4B**

## Study 2 - Validation *(N=245)*
### *Learning (N=147) / Test (N=98)*

| | Performance |
|---|---|
| AUC | **0.901** |
| CI 95% | [0.840 -0.959] |
| Se | **80.6%** |
| Sp | **85.1%** |
| PPV | 71.4% |
| NPV | 90.5% |
| Acc | 83.7% |
| Cutoff | 0.5 |

## Study 3 - Replication
### *External Validation (N=94)*

| | Performance |
|---|---|
| AUC | **0.906** |
| CI 95% | [0.832 – 0.964] |
| Se | **85.7%** |
| Sp | 80.3% |
| PPV | 64.9% |
| NPV | 92.9% |
| Acc | 81.9% |
| Cutoff | 0.5 |

Figures 5A

Figures 5B

**Figures 5A-5B**

**Figure 6**

Europäisches
Patentamt

European
Patent Office

Office européen
des brevets

# EUROPEAN SEARCH REPORT

Application Number

EP 22 21 5708

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2021/089865 A1 (ALCEDIAG [FR]) 14 May 2021 (2021-05-14) * the whole document * | 1-16 | INV. C12Q1/6883 |
| A,D | WO 2021/089866 A1 (ALCEDIAG [FR]) 14 May 2021 (2021-05-14) | 1-16 | |
| A | SALVETAT NICOLAS ET AL: "A game changer for bipolar disorder diagnosis using RNA editing-based biomarkers", TRANSLATIONAL PSYCHIATRY, vol. 12, no. 1, 4 May 2022 (2022-05-04), XP093082066, DOI: 10.1038/s41398-022-01938-6 Retrieved from the Internet: URL:https://www.nature.com/articles/s41398-022-01938-6> * the whole document * | 1-16 | |
| A,D | SCHNABL B ET AL: "Zinc finger protein 267 is up-regulated during the activation process of human hepatic stellate cells and functions as a negative transcriptional regulator of MMP-10", BIOCHEMICAL AND BIOPHYSICAL RESEARCH COMMUNICATIONS, ELSEVIER, AMSTERDAM NL, vol. 335, no. 1, 16 September 2005 (2005-09-16), pages 87-96, XP027452262, ISSN: 0006-291X, DOI: 10.1016/J.BBRC.2005.07.043 [retrieved on 2005-08-14] * page 88, left-hand column, paragraph 4 * | 15,16 | TECHNICAL FIELDS SEARCHED (IPC) C12Q |

The present search report has been drawn up for all claims

| Place of search | Date of completion of the search | Examiner |
|---|---|---|
| Munich | 14 September 2023 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 5708

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2023

| Patent document cited in search report | | Publication date | Patent family member(s) | | Publication date |
|---|---|---|---|---|---|
| WO 2021089865 | A1 | 14-05-2021 | BR 112022008886 | A2 | 23-08-2022 |
| | | | CA 3155714 | A1 | 14-05-2021 |
| | | | CN 114929898 | A | 19-08-2022 |
| | | | EP 3819386 | A1 | 12-05-2021 |
| | | | EP 4055190 | A1 | 14-09-2022 |
| | | | IL 292587 | A | 01-06-2022 |
| | | | JP 2023500845 | A | 11-01-2023 |
| | | | KR 20220097469 | A | 07-07-2022 |
| | | | WO 2021089865 | A1 | 14-05-2021 |
| WO 2021089866 | A1 | 14-05-2021 | CA 3155693 | A1 | 14-05-2021 |
| | | | CN 114945686 | A | 26-08-2022 |
| | | | EP 3819387 | A1 | 12-05-2021 |
| | | | EP 4055191 | A1 | 14-09-2022 |
| | | | IL 292588 | A | 01-06-2022 |
| | | | JP 2023500847 | A | 11-01-2023 |
| | | | KR 20220094201 | A | 05-07-2022 |
| | | | US 2023272472 | A1 | 31-08-2023 |
| | | | WO 2021089866 | A1 | 14-05-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021089865 A
- WO 2021089866 A
- WO 2021089866 A1

### Non-patent literature cited in the description

- **SALVETAT.** *Translational Psychiatry,* 2022, vol. 12, 182
- **JUDD LL ; AKISKAL HS ; SCHETTLER PJ ; ENDICOTT J ; MASER J ; SOLOMON DA et al.** The longterm natural history of the weekly symptomatic status of bipolar I disorder. *Arch Gen Psychiatry,* 2002, vol. 59, 530-7
- **JUNG Y ; GOLDMAN D.** Role of RNA modifications in brain and behavior. *Genes Brain Behav,* 2018, vol. 17, e12444
- **SHELTON RC.** The molecular neurobiology of depression. *Psychiatr Clin North Am,* 2007, vol. 30, 1-11
- **LEE SY ; LU RB ; WANG LJ ; CHANG CH ; LU T ; WANG TY et al.** Serum miRNA as a possible biomarker in the diagnosis of bipolar II disorder. *Sci Rep.,* 2020, vol. 10, 1131
- **GIACOPUZZI E ; GENNARELLI M ; SACCO C ; FILIPPINI A ; MINGARDI J ; MAGRI C et al.** Genome-wide analysis of consistently RNA edited sites in human blood reveals interactions with mRNA processing genes and suggests correlations with cell types and biological variables. *BMC Genomics,* 2018, vol. 19, 963
- **YANG W ; WANG Q ; KANES SJ ; MURRAY JM ; NISHIKURA K.** Altered RNA editing of serotonin 5-HT2C receptor induced by interferon: implications for depression associated with cytokine therapy. *Brain Res Mol Brain Res,* 2004, vol. 124, 70-8
- **LYDDON R ; DWORK AJ ; KEDDACHE M ; SIEVER LJ ; DRACHEVA S.** Serotonin 2c receptor RNA editing in major depression and suicide. *World J Biol Psychiatry,* 2013, vol. 14, 590-601
- **WEISSMANN D ; VAN DER LAAN S ; UNDERWOOD MD ; SALVETAT N ; CAVAREC L ; VINCENT L et al.** Region-specific alterations of A-to-I RNA editing of serotonin 2c receptor in the cortex of suicides with major depression. *Transl Psychiatry,* 2016, vol. 6, e878

- **CHIMIENTI F ; CAVAREC L ; VINCENT L ; SALVETAT N ; ARANGO V ; UNDERWOOD MD et al.** Brain region-specific alterations of RNA editing in PDE8A mRNA in suicide decedents. *Transl Psychiatry,* 2019, vol. 9, 91
- **SALVETAT N ; VAN DER LAAN S ; VIRE B ; CHIMIENTI F ; CLEOPHAX S ; BRONOWICKI JP et al.** RNA editing blood biomarkers for predicting mood alterations in HCV patients. *J Neurovirol,* 2019, vol. 25, 825-36
- **SALVETAT N ; CHIMIENTI F ; CAYZAC C ; DUBUC B ; CHECA-ROBLES F ; DUPRE P et al.** Phosphodiesterase 8A to discriminate in blood samples depressed patients and suicide attempters from healthy controls based on A-to-I RNA editing modifications. *Transl Psychiatry,* 2021, vol. 11, 255
- **SALVETAT N ; CHECA-ROBLES FJ ; PATEL V ; CAYZAC C ; DUBUC B ; CHIMIENTI F ; ABRAHAM JD ; DUPRÉ P ; VETTER D ; MÉREUZE S.** A game changer for bipolar disorder diagnosis using RNA editing-based biomarkers. *Transl Psychiatry,* 04 May 2022, vol. 12 (1), 182
- **BUENO-NOTIVOL J ; GRACIA-GARCÍA P ; OLAYA B ; LASHERAS I ; LÓPEZ-ANTÓN R ; SANTABÁRBARA J.** Prevalence of depression during the COVID-19 outbreak: a metaanalysis of community-based studies. *Int J Clin Health Psychol,* 2021, vol. 21, 100196
- **WU T ; JIA X ; SHI H ; NIU J ; YIN X ; XIE J et al.** Prevalence of mental health problems during the COVID-19 pandemic: A systematic review and meta-analysis. *J Affect Disord,* 2021, vol. 281, 91-98
- **SCHNABL B ; HU K ; MUHLBAUER M ; HELLERBRAND C ; STEFANOVIC B ; BRENNER DA et al.** Zinc finger protein 267 is up-regulated during the activation process of human hepatic stellate cells and functions as a negative transcriptional regulator of MMP-10. *Biochemical and biophysical research communications,* 2005, vol. 335, 87-96

- **SCHNABL B ; VALLETTA D ; KIROVSKI G ; HELLERBRAND C.** Zinc finger protein 267 is up-regulated in hepatocellular carcinoma and promotes tumor cell proliferation and migration. *Experimental and molecular pathology,* 2011, vol. 91, 695-701
- **SCHNABL B ; CZECH B ; VALLETTA D ; WEISS TS ; KIROVSKI G ; HELLERBRAND C.** Increased expression of zinc finger protein 267 in non-alcoholic fatty liver disease. *International journal of clinical and experimental pathology,* 2011, vol. 4, 661-666
- **LU L ; CHEN XM ; TAO HM et al.** Regulation of the expression of zinc finger protein genes by microRNAs enriched within acute lymphoblastic leukemia-derived microvesicles. *Genet Mol Res,* 2015, vol. 14 (4), 11884-1189
- **CHEISHVILI D ; PARASHAR S ; MAHMOOD N ; ARAKELIAN A ; KREMER R ; GOLTZMAN D et al.** Identification of an Epigenetic Signature of Osteoporosis in Blood DNA of Postmenopausal Women. *Journal of bone and mineral research : the official journal of the American Society for Bone and Mineral Research,* 2018, vol. 33, 1980-1989
- **PATEL S ; HOWARD D ; CHOWDHURY N et al.** Characterization of human genes modulated by por-phyromonas gingivalis highlights the ribosome, hypothalamus, and cholinergic neurons. *Front Immunol,* 2021, vol. 12, 646259
- **FEHLBAUM-BEURDELEY P ; SOL O ; DÉSIRÉ L ; TOUCHON J ; DANTOINE T ; VERCELLETTO M et al.** Validation of AclarusDxTM, A Blood-Based Transcriptomic Signature for the Diagnosis of Alzheimer's Disease. *J Alzheimers Dis,* 2012, vol. 32, 169-81
- **NGUYEN HD ; JO WH ; HOANG NHM ; KIM MS.** Curcumin-Attenuated TREM-1/DAP12/NLRP3/Caspase-1/IL1B, TLR4/NF-κB Pathways, and Tau Hyperphosphorylation Induced by 1,2-Diacetyl Benzene: An in Vitro and in Silico Study. *Neurotox Res,* October 2022, vol. 40 (5), 1272-1291
- **PIERRE GEURTS ; DAMIEN ERNST ; LOUIS WEHENKEL.** Extremely randomized trees. *Mach Learn,* 2006, vol. 63, 3-42